(19) Europäisches Patentamt

European Patent Office

Office européen des brevets

(11) **EP 1 493 812 A2**

(12) **EUROPEAN PATENT APPLICATION**

(43) Date of publication:
**05.01.2005 Bulletin 2005/01**

(51) Int Cl.⁷: **C12N 15/12**, C07K 14/50,
A61K 38/18, C07K 16/24

(21) Application number: **04023638.2**

(22) Date of filing: **28.04.1994**

(84) Designated Contracting States:
**AT BE CH DE DK ES FR GB GR IE IT LI LU MC NL PT SE**

(30) Priority: **29.06.1993 US 86427**

(62) Document number(s) of the earlier application(s) in accordance with Art. 76 EPC:
**94916597.1 / 0 706 563**

(71) Applicant: **CHIRON CORPORATION
Emeryville California 94608-2916 (US)**

(72) Inventors:
  • **Gospodarowicz, Denis J
  Lafayette, CA 94549 (US)**

  • **Masiarz, Frank
  San Francisco, CA 94131 (US)**

(74) Representative: **Woods, Geoffrey Corlett
J.A. KEMP & CO.
Gray's Inn
14 South Square
London WC1R 5JJ (GB)**

Remarks:
This application was filed on 05 - 10 - 2004 as a divisional application to the application mentioned under INID code 62.

(54) **A truncated keratinocyte growth factor (KGF) having increased biological activity**

(57) The present invention provides an isolated polypeptide, wherein said polypeptide is (a) a fragment of mature, full-length, human keratinocyte growth-factor (KGF) as depicted in Figure 1 or (b) an analog of said fragment, wherein said polypeptide exhibits an increase in bioactivity relative to said mature, full-length, human KGF as determined by the Balb/MK bioactivity assay.

**EP 1 493 812 A2**

## Description

### FIELD OF THE INVENTION

The present invention relates generally to keratinocyte growth factor ("KGF"). More specifically, this invention relates to a truncated KGF fragment and analogs thereof having increased biological activity and decreased cytotoxicity as compared to a recombinant, full length, KGF expressed in an insect cell system. This KGF fragment, designated herein as $KGF_{des1-23}$ lacks the first 23 amino acid residues of the N-terminus of mature full-length KGF, this N-terminus that includes a glycosylation site at amino acid residues 14 to 16 from the N-terminus as indicated in Finch, P.W. *et al*. *Science* 245:752-755 (1989) and was previously believed to confer upon KGF its epithelial cell specificity.

### BACKGROUND OF THE INVENTION

KGF belongs to a family of fibroblast growth factors ("FGFs"), the prototype of which is represented by basic FGF ("bFGF"). KGF is, hence, also known as FGF-6. Like other FGFs, KGF is a heparin binding protein, but unlike other FGFs, it has a unique target cell specificity. In particular, FGFs are generally capable of stimulating the proliferation and differentiation of a variety of cell types derived from the primary or secondary mesoderm as well as from neuroectoderm. KGF is similar to other FGFs in its ability to stimulate epithelial cell proliferation, but is dissimilar to other FGFs in its inability to stimulate endothelial cells or fibroblast proliferation, as discussed in Finch, P.W. *et al*. (*loc. cit.*)

FGFs, including acidic fibroblast growth factor ("aFGF") and basic fibroblast growth factor ("bFGF"), are known to have heparin-binding properties and have the ability to induce the differentiation and proliferation of ventral, as well as dorsal mesoderm in early blastulae, as discussed in Gospodarowicz *et al*., *Cell. Biol. Rev.* 25:307-314 (1991), and Basilico *et al*., *Adv. Cancer Res.* 59:115-165 (1992). The response of cells to FGF is mediated through binding thereof to cell surface receptors ("FGFRs"), of which there are three inter-related types, as discussed in Hou *et al*., *Science* 251:665-668 (1991). High affinity FGFRs are tyrosine kinases and include the *flg* receptor ("FGFR-1"), the *bek* receptor ("FGFR-2"), and the *K Sam* receptor ("FGFR-3"), as discussed in Lee *et al*., *Science* 245:57-60 (1989); Dionne *et al*., *EMBO 9*:2685-2692 (1990); Miki *et al*., *Science 251*:72-75 (1991); Miki *et al*., *Proc. Natl. Acad. Sci. USA 89*: 246-250 (1992); and Dell *et al*., *J. Biol. Chem. 267*: 21225-21229 (1992).

Both FGFR-1 and FGFR-2 are widely expressed in mesodermal and neuroendodermal tissues, and both are able to bind aFGF and bFGF with similar affinities. FGFR-3 is a KGF receptor that is specific to epithelial cells. It is an alternative transcript of FGFR-2. In contrast to FGFR-2, which shows high affinity for both aFGF and bFGF and no affinity for KGF, FGFR-3 binds KGF and aFGF with an affinity approximately 20 to 1000 fold higher than bFGF, as discussed in Miki *et al*. (*loc. cit*.), and Dell *et al*. (*loc. cit.*)

The tightly restricted activity profile of KGF to epithelial cells is desirable, for example, as in many types of wound healing applications as well as in the treatment of hyperproliferative diseases of the epidermis such as psoriasis and basal cell carcinoma. Presently, except for KGF, no highly suitable mitogenic factor exists for these applications. It would be desirable, therefore, if KGF could be modified to increase its potency and decrease its cytotoxicity, for therapeutic applications.

Recently, Ron *et al*., *J. Biol. Chem. 268*: 2984-2988 (February 1993) found that when $KGF_{163}$ was expressed in a T7 prokaryotic expression system, a recombinant KGF ("rKGF") polypeptide could be obtained that possessed mitogenic activity. When the rKGF molecule was truncated by deletion of 3, 8, 27, 38, and 48 amino acid residues, respectively, from the N-terminus of the mature $KGF_{163}$ polypeptide, biological activity of the resulting molecules varied. With deletion of 3 and 8 amino acid residues, respectively, the mitogenic activity of the resulting molecules did not appear to be affected as compared to full-length rKGF ("$rKGF_{163}$"). Deletion of 27 amino acid residues, however, resulted in 10-20 fold reduced mitogenic activity. Deletion of 38 and 48 amino acid residues, respectively, resulted in complete loss of mitogenic activity and heparin binding ability. Thus, Ron *et al*. failed to produce any truncated KGF fragments that possessed increased mitogenic activity as compared to the $rKGF_{163}$ molecule.

### SUMMARY OF THE INVENTION

One of the objects of the present invention is to provide a KGF fragment that contains a portion of an amino acid sequence of mature, full-length KGF, $KGF_{163}$, and possesses at least a 2-fold increase in mitogenic activity as compared to the full-length recombinant KGF, $rKGF_{163}$ A particular objective is to provide a KGF fragment that lacks a sequence comprising the first 23 N-terminus amino acid residues, C-N-D-M-T-P-E-Q-M-A-T-N-V-N-C-S-S-P-E-R-H-T-R-, of $rKGF_{163}$.

Another one of the objects of the present invention is to provide a KGF fragment that has decreased cytotoxicity as compared to $rKGF_{163}$. Still another object is to provide a conjugate that comprises the KGF fragment described above and a toxin molecule. The toxin molecule can be at least one of ricin A, diphtheria toxin, and saporin.

Yet another one of the objects of the present invention is to provide a therapeutic composition that contains the KGF fragment, as described above, and a pharmaceutically acceptable carrier, for example, one suitable for topical application to human skin.

Still another one of the objects of the present invention is to provide a DNA molecule that is composed of a nucleotide sequence that encodes the KGF fragment described above.

Yet another one of the objects of the present invention is to provide an expression vector that contains the DNA molecule that encodes the KGF fragment, described above, and a regulatory sequence for expression of the DNA molecule. The expression vector can be, for example, a baculovirus.

Yet another one of the objects of the present invention is to provide a host cell transformed with the expression vector described above. The host cell can be, for example, a bacterial cell, a yeast cell, a mammalian cell, or an insect cell.

Yet another one of the objects of the present invention is to provide a method of producing the KGF fragment by culturing the transformed host cell, as described above, and isolating the KGF fragment from the culture.

Still another one of the objects of the present invention is to provide a method of stimulating epithelial cell growth by applying the KGF fragment to an area in which epithelial cell growth is desired and allowing the cell to grow.

Still another one of the objects of the present invention is to provide a method for wound healing by applying the therapeutic composition described above to an area of a wound to be treated and allowing the wound to heal.

Still another one of the objects of the present invention is to provide a method of treating a hyperproliferative disease of the epidermis by applying the conjugate described above to an area to be treated.

Further objects, features, and advantages of the present invention would be apparent to a person of ordinary skill in the art and need not be enumerated here. The present invention includes variants and modifications of the above that are apparent to a person of ordinary skill in the art and that do not substantially alter the nature and activity of the fragment, conjugate, therapeutic composition, vector, host, and methods of use.

## SUMMARY OF THE DRAWINGS

FIG. 1 shows the amino acid sequence of the mature, full-length human KGF polypeptide, beginning with the N-terminus and containing 163 amino acid residues and a single N-linked glycosylation signal at amino acid residues 14-16.

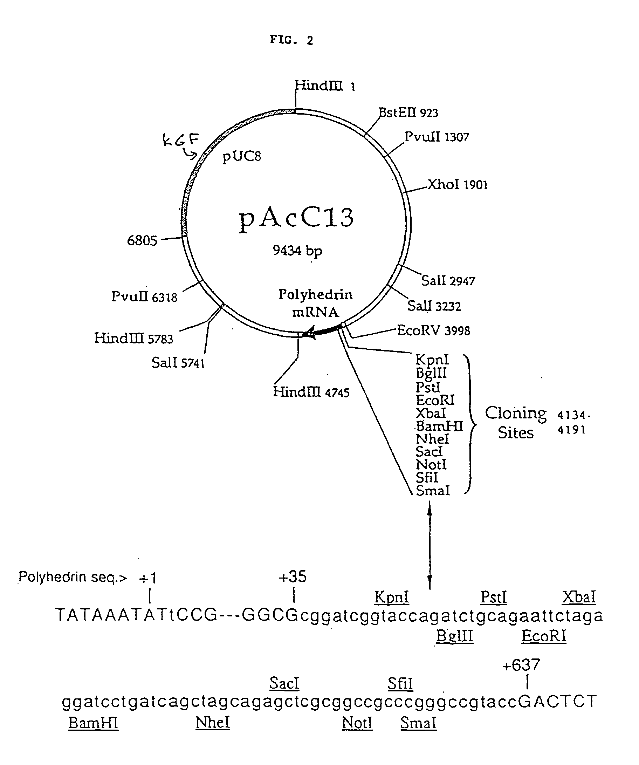

FIG. 2 shows the pAcC13 expression vector into which the 163 amino acid sequence of KGF has been inserted.

FIG. 3 illustrates the further purification of the KGF fragment of the present invention from the SF9 cells conditioned medium after elution in a Heparin Sepharose ("HS") column with 1 M NaCl. The bioactive fractions from the HS column were pooled, diluted 5-fold with 10 mM Tris, pH7.3, and loaded onto a Mono S HR5/5 cation exchange FPLC.

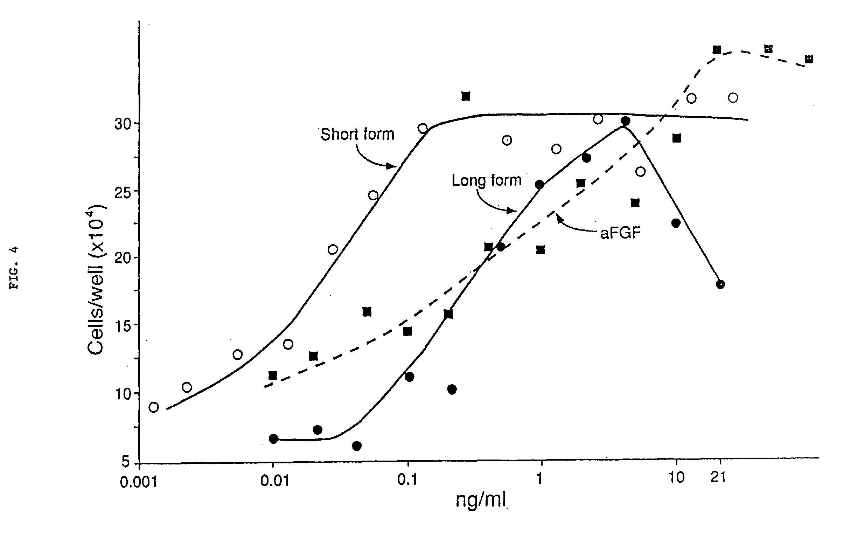

FIG. 4 compares the biological activity of the long, i.e., $rKGF_{163}$, and the short, i.e., $KGF_{des1-23}$, form of KGF versus aFGF on cells of the Balb/Mk cell line.

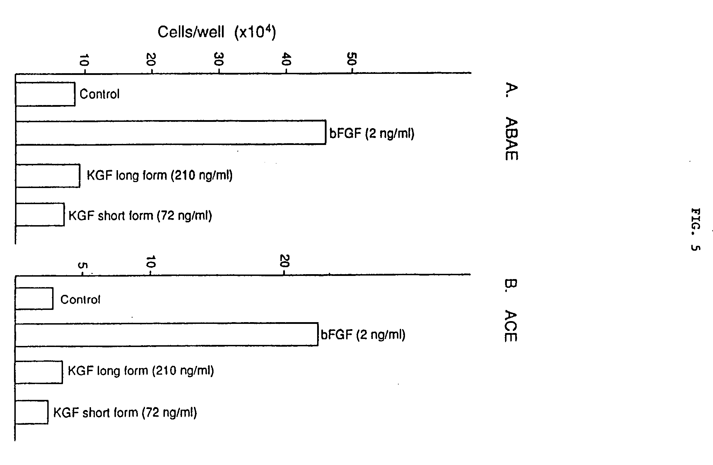

FIG. 5 compares the biological activity of the long, i.e., $rKGF_{163}$, and the short, i.e., $KGF_{des1-23}$, form of KGF versus bFGF on vascular endothelial cells derived from large vessels (A:ABAE cells) or capillary (B:ACE cells).

## DETAILED DESCRIPTION OF THE PREFERRED EMBODIMENTS

It has been surprisingly discovered that a truncated, unglycosylated KGF, referred to herein as the KGF fragment or $KGF_{des1-23}$, having a deletion spanning the first 23 N-terminus amino acid residues of $rKGF_{163}$, possesses a greater biological activity and decreased cytotoxicity on epithelial cells as compared with the $rKGF_{163}$. Generally, the KGF fragment of the present invention retains the specificity of $KGF_{163}$ for stimulation of epithelial cell proliferation.

Accordingly, a preferred embodiment of the present invention is a novel truncated, unglycosylated KGF fragment, $KGF_{des1-23}$, unaccompanied by impurities that normally accompany the native molecule when it is produced *in vivo*. This fragment has an apparent molecular weight of about 18 kDa based upon its migration in Na Dod $SO_4$/PAGE as a single peak. The specific activity of purified $KGF_{des1-23}$ on Balb/Mk cells is approximately 2.5 x $10^7$ units per milligram ($ED_{50}$ 40 pg/ml), about 7- to 10-fold greater than that of the $rKGF_{163}$ protein, or aFGF when compared in a cell proliferation assay, and 100-fold greater than when $rKGF_{163}$ is bioassayed by examining initiation of DNA synthesis in Balb/Mk cells in a chemically defined medium, as described in PCT patent application no. WO 90/08771.

In another preferred embodiment of the present invention, $KGF_{des-1-23}$ is produced by recombinant DNA technology, particularly for large-scale commercial production. In a further preferred embodiment, recombinant DNA molecule and an expression vector each containing $KGF_{des1-23}$ or analogs thereof in accordance with the present invention is carried out by standard gene expression technology using methods well known in the art.

In a further embodiment, the DNA or vector comprising the DNA encoding $KGF_{des1-23}$ can be expressed in a bacterial, mammalian, yeast, or insect cell expression system. In a preferred embodiment, a bacterial or yeast cell expression system is ideal for production of the $KGF_{des1-23}$ fragment. In another preferred embodiment, the DNA or vector is expressed in an insect cell expression system.

The KGF fragment of the present invention can be used for identification of receptor recognition sites as well as for the design of peptide agonists or antagonists. Moreover, in view of the unique specificity of KGF for keratinocytes, its inability to induce the proliferation of vascular endothelial cells or fibroblasts, its and lack of cytotoxicity, in another embodiment of the present invention, KGF$_{des1-23}$ is preferably used for wound healing applications, particularly where there is a desire to promote re-epithelialization of the skin. A preferred embodiment, KGF$_{des1-23}$ is used in corneal epithelial repair. Other applications of KGF$_{des1-23}$ can be envisaged based upon its specificity for epithelial cells found in the gastrointestinal tract.

The selection of the KGF fragment herein over other growth factors such as epidermal growth factor ("EGF"), platelet-derived growth factor ("PDGF"), and other FGFs for skin repair is within the skill of a person in the art. The other growth factors, for example, induce fibroplasia and angiogenesis, in addition to stimulating either directly or indirectly keratinocyte proliferation. In skin repair, such additional activities could produce undesirable side effects such as scaring. In corneal repair involving either a wound or surgery, the use of these other factors could induce blood vessel invasion into the cornea, resulting in undesirable cornea opacity or edema. KGF, on the other hand, has a unique specificity for keratinocytes and does not induce the proliferation of vascular endothelial cells or fibroblasts and, therefore, would be the agent of choice for these particular wound healing applications.

Further, the KGF fragment of the present invention can be used in another embodiment of the present invention, in the form of a KGF fragment/toxin conjugate. Such a conjugate can slow down or stop the disease process in diseases such as psoriasis that results from hyperproliferation of the basal layer of keratinocytes from the epidermis. This conjugate would not affect the other two major celltypes such as vascular endothelial cells and fibroblasts, present in these tissues.

In another embodiment of the present invention a KGF fragment/toxin conjugate may be used to eradicate tumor cell proliferation, for example, basal cell carcinoma.

The practice of the present invention will employ, unless otherwise indicated, conventional techniques of molecular biology, microbiology, recombinant DNA, and immunology, that are within the skill of the art. Such techniques are explained fully in the literature. *See* e.g., Sambrook, et al., MOLECULAR CLONING: A LABORATORY MANUAL, 2nd ed. Cold Spring Harbor Laboratory Press (1989); DNA CLONING, VOLUMES I AND II, D.N Glover ed. IRL Press (1985); OLIGONUCLEOTIDE SYNTHESIS M.J. Gait ed., IRL Press (1984); NUCLEIC ACID HYBRIDIZATION, B.D. Hames & S.J. Higgins eds. IRL Press (1984); TRANSCRIPTION AND TRANSLATION, B.D. Hames & S.J. Higgins eds. IRL Press (1984); ANIMAL CELL CULTURE, R.I. Freshney ed., IRL Press (1986); B. Perbal, A PRACTICAL GUIDE TO MOLECULAR CLONING, (1984); the series, METHODS IN ENZYMOLOGY, Academic Press, Inc.; GENE TRANSFER VECTORS FOR MAMMALIAN CELLS, J.H. Miller and M.P. Calos eds., Cold Spring Harbor Laboratory (1987); METHODS IN ENZYMOLOGY, Vol. 154 and Vol. 155, Wu and Grossman, and Wu, eds., respectively, [Mayer and Walker, eds. (1987), IMMUNOCHEMICAL METHODS IN CELL AND MOLECULAR BIOLOGY, Academic Press, London, Scopes , (1987), PROTEIN PURIFICATION: PRINCIPLES AND PRACTICE, 2nd ed. (Springer-Verlag, N. Y. ), and HANDBOOK OF EXPERIMENTAL IMMUNOLOGY, VOLUMES I-IV, D.M. Weir and C. C. Blackwell eds., (1986); Kitts *et al.*, *Biotechniques* 14:810-817 (1993); Munemitsu *et al.*, *Mol. and Cell. Biol.* 10: 5977-5982 (1990).

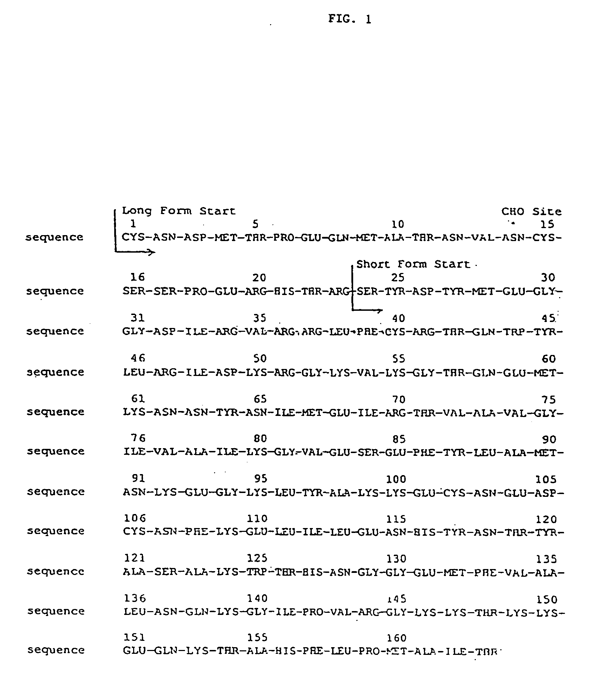

Standard abbreviations for amino acids are used in Fig. 1 and elsewhere in this specification. All publications, patents, and patent applications cited herein are incorporated by reference. For clarification, the terms used herein are more particularly defined below.

Definitions

As used herein, the term "keratinocyte growth factor" or "KGF" refers to a polypeptide that belongs to a family of structurally distinct proteins, the fibroblast growth factors, FGFs, that display varying degrees of sequence homology suggesting that they are encoded by a related family of genes. KGF has the characteristics described elsewhere in this specification, for example, it binds to FGFR-3 and is capable of stimulating growth of epithelial cells, especially keratinocytes. Full-length KGF is comprised of 163 amino acid residues.

As used herein, the KGF fragment of the present invention is a polypeptide that is composed of a portion of the amino acid sequence of the mature, full-length KGF. An analog of the KGF fragment, as used herein, includes minor insertions, deletions or substitutions of the KGF fragment that do not substantially affect its properties. For example, conservative amino acid replacements are contemplated. Such replacements are, for example, those that take place within a family of amino acids that are related in their side chains. The families of amino acids include (1) acidic: aspartic acid, glutamic acid; (2) basic: lysine, arginine, histidine; (3) non-polar: alanine, valine, leucine, isoleucine, proline, phenylalanine, methionine, tryptophan; and (4) uncharged polar: glycine, asparagine, glutamine, cystine, serine, threonine, tyrosine. Phenylalanine, tryptophan, and tyrosine are sometimes classified jointly as a family of aromatic amino acids. In particular, it is generally accepted that conservative amino acid replacements consisting of an isolated replacement of a leucine with an isoleucine or valine, or an aspartic acid with a glutamic acid, or a threonine with a serine, or a similar conservative replacement of an amino acid with a structurally related amino acid, in an area outside of the polypeptide's active site will not have a major effect on the properties of the polypeptide.

The properties of the KGF fragment include (i) its biological activity such as a 2-10 fold, preferably a 2 fold, most preferably a 7-10 fold increase in stimulation of epithelial cell proliferation as compared to the mature, full-length native KGF molecule, and (ii) its ability to bind to FGFR-3.

The term "recombinant polynucleotide" as used herein intends a polynucleotide of semisynthetic, or synthetic origin, or encoded by cDNA or genomic DNA ("gDNA") such that (1) it is not associated with all or a portion of a polynucleotide with which it is associated in nature, (2) is linked to a polynucleotide other than that to which it is linked in nature, or (3) does not occur in nature.

A "replicon" is any genetic element, such as a plasmid, a chromosome, a virus, a cosmid, etc., that behaves as an autonomous unit of polynucleotide replication within a cell; i.e., it is capable of replication under its own control. A replicon may include, for example selectable markers.

A "recombinant vector" is a replicon in which another polynucleotide segment is attached, so as to bring about the replication and/or expression of the attached polynucleotide segment.

A "regulatory sequence" refers to a polynucleotide sequence that is necessary for regulation of expression of a coding sequence to which the polynucleotide sequence is operably linked. The nature of such regulatory sequences differs depending upon the host cell in which the coding sequence is to be expressed. In prokaryotes, such regulatory sequences generally include, for example, a promoter, a ribosomal binding site, and/or a transcription termination sequence. In eukaryotes, generally, such regulatory sequences include a promoter and/or a transcription termination sequence. The term "regulatory sequence" may also include additional components the presence of which is advantageous, for example, a secretory leader sequence that encodes for secretion of the polypeptide to be expressed.

"Operably linked" refers to a juxtaposition wherein the components so linked are in a relationship permitting them to function in their intended manner. For example, a regulatory sequence is "operably linked" to a coding sequence when it is joined in such a way that expression of the coding sequence is achieved under conditions compatible with the regulatory sequence.

A "coding sequence" is a polynucleotide sequence that is translated into a polypeptide, when placed under the control of an appropriate regulatory sequence. The boundaries of the coding sequence are generally determined by a translation start codon at its 5'-terminus and a translation stop codon at its 3'-terminus. A coding sequence can include, for example, cDNA, and recombinant polynucleotide sequences.

"PCR" refers to the technique of polymerase chain reaction as described in Saiki, *et al.*, *Nature* 324: 163 (1986); and Scharf *et al.*, *Science* 233:1076-1078 (1986); U.S. Patent No. 4,683,195; and U.S. Patent No. 4,683,202.

As used herein, the term "polypeptide" refers to a polymer of amino acids and does not refer to a specific length of the product. Thus, peptides, oligopeptides, and proteins are included in this term. This term also includes translation modifications of the polypeptide, for example, glycosylations, acetylations, phosphorylations and the like. Further included in this definition are, for example, polypeptides with substituted linkages, as well as other modifications known in the art, both naturally occurring and non-naturally occurring.

As used herein, "terminators" are regulatory sequences, such as polyadenylation and transcription termination sequences, located 3' or downstream of the stop codon of the coding sequences.

"Recombinant host cells," "host cells," "cells," "cell cultures," and other such terms denote, for example, microorganisms, insect cells, and mammalian cells, that can be or have been used as recipients for introduction of recombinant vector or other transfer DNA, and include the progeny of the cell that has been transformed. Such progenies include those that may not necessarily be identical in morphology or in genomic or total DNA complement as the original parent, and that may be produced as a result of natural, accidental, or deliberate mutation. Examples of mammalian host cells include Chinese hamster ovary ("CHO") and monkey kidney ("COS") cells.

As used herein, the term "microorganism" includes prokaryotic and eukaryotic microbial species such as bacteria and fungi, the latter including yeast and filamentous fungi.

"Transformation," as used herein, refers to the transfer of an exogenous polynucleotide into a host cell, irrespective of the method used for the transfer, which can be, for example, by infection, direct uptake, transduction, F-mating, microinjection or electroporation. The exogenous polynucleotide may be maintained as a non-integrated vector, for example, a plasmid, or alternatively, may be integrated into the host genome.

"Purified" and "isolated" in reference to a polypeptide or a nucleotide sequence means that the indicated molecule is present in substantial absence of other biological macromolecules of the same species or type. The term "purified", as used herein means at least 75% by weight; preferably, at least 85% by weight, more preferably, at least 95% by weight and, most preferably, at least 98% by weight, of biological macromolecules of the same type are present, but water, buffers, and other small molecules, especially molecules having a molecular weight of less than 1000, can be present as well.

By "pharmaceutically acceptable carrier," is meant any carrier that is used by persons in the art for a administration into a human that does not itself induce any undesirable side effects such as the production of antibodies, fever, etc. Suitable carriers are typically large, slowly metabolized macromolecules that can be a protein, a polysaccharide, a polylactic acid, a polyglycolic acid, a polymeric amino acid, amino acid copolymers or an inactive virus particle. Other carriers are well known to those of ordinary skill in the art. Preferably, the carrier contains thyroglobulin.

A therapeutic composition typically will contain pharmaceutically acceptable carriers, such as water, saline, glycerol, ethanol, etc. Additionally, auxiliary substances, such as wetting or emulsifying agents, pH buffering substances, and the like, may be present in such compositions.

By "A therapeutically effective amount," as used herein refers to that amount that is effective for production of a desired result. This amount varies depending upon the health and physical condition of the individual to be treated, the capacity of the individual's immune system to synthesize antibodies, the degree of protection desired, the formulation, the treating doctor's assessment of the medical situation, and other relevant factors. It is expected that the amount will fall in a relatively broad range that can be determined through routine trials.

It has been unexpectedly found that when KGF is expressed in insect cells, *Spodoptera frugiperda* ("SF9"), by infection of a recombinant baculovirus, *Autographa californica*, containing the cDNA encoding $KGF_{163}$, they produced a KGF fragment that lacks the first 23 amino acid residues of the $KGF_{163}$ N-terminal domain containing a single glycosylation site, in addition to $KGF_{163}$. The truncated and unglycosylated KGF fragment $KGF_{des1-23}$ or $KGF_{140}$, in contrast to the native long form identified as $KGF_{163}$, has a 7- to 10-fold increased potency on target cells. The target cell specificity of $KGF_{des1-23}$ is unchanged. Additionally, at high concentrations of the KGF fragment, no toxic effect on keratinocytes is observed in contrast to that observed for $KGF_{163}$. These observations suggest that contrary to what was previously proposed in PCT application no. WO 90/08771, the target cell specificity of KGF does not reside in its N-terminal domain. Furthermore, the present invention shows that an N-terminally truncated version of KGF in fact represents an improved KGF version with higher biological activity and decreased cytotoxicity for therapeutic application.

A large amount of the KGF fragment can be produced by recombinant DNA techniques which is preferable over the alternative process of isolating and purifying KGF from natural sources and cleaving off the first 23 amino acid residues from the N-terminal thereof. KGF produced by recombinant techniques permits the KGF to be isolated in the absence of contaminating molecules that are normally present in cells. Indeed, KGF compositions entirely free of any trace of human protein contaminants can readily be produced in, for example, bacterial cells, yeast cells, and insect cells. By use of recombinant DNA techniques, KGF fragments that are not found in nature, such as variants that can be produced by site-directed mutagenesis, can also be produced.

Any promoter that would allow expression of the KGF fragment in the desired host can be used in the present invention. For example, in *E. coli,* the regulatory promoter sequence of the *lac* operon can be used. Another example is the yeast alcohol dehydrogenase ("ADH") promoter, which has an upstream activator sequence ("UAS") that modulates the activity of the ADH promotor. Additionally, certain viral enhancers can also be used herein. Such enhancers not only amplify but also regulate expression in mammalian cells. These enhancers can be incorporated into mammalian promoter sequences which will become activated only in the presence of an inducer, such as a hormone or an enzyme substrate as discussed in Sassone-Corsi and Borelli *Trends Genet.* 2:215 (1986); Maniatis *et al. Science* 236: 1237 (1987). Promotors that may be used in the present invention also include the baculovirus polyhedron hybrid promotor and the p10 promoter.

Examples of termination sequences that can be used in the present invention are the *Saccharomyces cerevisiae* alpha-factor terminator and the baculovirus terminator. Furthermore, viral terminators that are operable in certain host cells can also be used. For instance, the SV40 terminator is functional in Chinese Hamster Ovary (CHO) cells.

When designing truncated proteins, several of the KGFs are preferred, in the practice of this invention. One is unglycosylated KGF, for example, truncating the first 23 amino acids of native KGF. A cDNA encoding a preferred truncated KGF is presented in Figure 1. Other species of KGF fragments exist or can be created by routine methods. In the sequence shown, the cleavage site for a truncated protein may occur where indicated by arrow (a), resulting in a protein having a molecular weight of about 18,000 Da. Utilizing the sequence data in Figure 1, as well as the denoted characteristics of truncated KGF, it is within the skill of the art to obtain other DNA sequences encoding truncated KGF. For example, the structural gene may be manipulated by varying individual nucleotides, while retaining the correct amino acid(s), or varying the nucleotides, so as to modify the amino acids, without loss of biological activity. Nucleotides may be substituted, inserted, or deleted by known techniques, including, for example, in vitro mutagenesis and primer repair. The structural gene may be truncated at its 3'-terminus and/or its 5'-terminus while retaining its biological activity.

The KGF fragment coding sequence can be constructed by synthesizing the DNA sequence encoding the KGF fragment or by altering an existing or native KGF coding sequence to produce the desired sequence. The native KGF coding sequence or polypeptide are those that occur in nature. The amino acid sequence of the native KGF is shown in FIGURE 1. The synthetic KGF fragment can be made based on the known amino acid sequence of KGF using codons preferred by the selected host cell as suggested in Urdea *et al.*, *Proc. Natl. Acad. Sci. USA 80*: 7461 (1983). Alternatively, the desired KGF fragment coding sequence can be cloned from nucleic acid libraries using probes based on the nucleic acid sequence shown in FIGURE 1. Techniques for producing and probing nucleic acid sequence libraries are described, for example, in Sambrook *et al.*, "Molecular Cloning: A Laboratory Manual" (New York, Cold Spring Harbor Laboratory, 1989). Other recombinant techniques, such as site specific mutagenesis, PCR, enzymatic digestion and ligation, can also be used to construct analogs and derivatives of the coding sequence of the KGF fragment. The native KGF polypeptide coding sequence can be easily modified to create the other classes of KGF polypeptides.

As an example, analogs of the KGF fragment can be created by conservative amino acid substitutions that do not impair the activity of the KGF fragment. The present invention further contemplates a subset of analogs of the KGF fragment called muteins, in which non-disulfide bond participating cysteines of the KGF fragment are substituted, generally, with serines. These muteins may be stable over a broader temperature range than the unmodified KGF fragment. The muteins herein can also contain amino acid deletions compared to the native KGF fragment. Muteins will retain at least 20%, preferably at least 50%, and most preferably at least 80% of the activity of the native KGF fragment. The coding sequence of muteins can be constructed by *in vitro* mutagenesis of the coding sequence of the native KGF fragment.

Fragments differ from the native KGF molecule by amino and/or carboxyl terminal amino acid deletions. The number of amino acids that are truncated is not critical as long as the KGF fragment does not contain the N-terminal glycosylation site and retains at least 20% of the KGF activity of the native KGF molecule. The coding sequence of such fragments can be easily constructed by cleaving the unwanted nucleotides from the native KGF coding sequence or a variant thereof.

An expression vector within the present invention contains a promoter that is operable in the host cell and is operably linked to the coding sequence of the KGF fragment or analog. An expression vector may optionally include a signal sequence for secretion, a terminator, a selectable marker, an origin of replication, and sequences homologous to host cell sequences. These additional elements can be included to optimize expression.

Functional non-natural promoters may also be used, for example, synthetic promoters based on a consensus sequence of different promoters. Also, effective promoters can be hybrid promoters that contain a regulatory region linked with a heterologous expression initiation region. Examples of hybrid promoters are the *E. coli* lac operator linked to the *E. coli* tac transcription activation region; the yeast alcohol dehydrogenase ("ADH") regulatory sequence linked to the yeast glyceraldehyde-3-phosphate-dehydrogenase ("GAPDH") transcription activation region as described in U.S. Patent Nos. 4,876,197 and 4,880,734, incorporated herein by reference; and the cytomegalovirus ("CMV") enhancer linked to the simian virus SV40 promoter.

The coding sequence for the KGF fragment or analog of the present invention may also be linked in reading frame to a signal sequence. The signal sequence typically encodes an amino acid sequence for secretion comprised of hydrophobic amino acids that direct the KGF fragment or analog to the cell membrane. In a preferred embodiment, a processing site is located between the signal sequence and the KGF fragment, to allow cleavage between the signal sequence and the KGF fragment either *in vivo* or *in vitro*. Suitable signal sequences are those derived from genes for secreted endogenous host cell proteins, such as the yeast invertase gene as described in European Patent No. 12873 and Japanese Patent No. 62,096,086, the A-factor gene as described in U.S. Patent No. 4,588,684, and interferon signal sequence as described in EP 60057.

A preferred class of secretion leaders for use in the present invention for yeast expression is the truncated yeast alpha-factor leader sequence, which contains at least a portion of the "pre" signal sequence, and the "pro" region. The alpha-factor leader sequences that can be employed herein include the full-length pre-pro alpha factor leader having about 83 amino acid residues, as well as truncated alpha-factor leaders, typically about 25 to about 50 amino acid residues, as described in U.S. Patent Nos. 4,546,083 and 4,870,008, and European Patent No. EP 324274 incorporated herein by reference. Additional leaders employing an alpha-factor leader fragment that can be used herein include hybrid alpha-factor leaders made with a presequence of a first yeast signal sequence, but a pro-region from a second yeast alpha-factor. (See *e.g.*, PCT WO 89/02463.)

f. Expression systems

KGF fragments and analogs thereof can be expressed by recombinant techniques when a DNA sequence encoding the KGF fragment or analog is operably linked into a vector in proper reading frame and orientation, as is well understood by those skilled in the art. When producing a genetic construct containing the KGF fragment, a preferred starting material is cDNA encoding the KGF fragment. Typically, the truncated KGF gene will be inserted downstream from a promoter and will be followed by a terminator sequence although the KGF fragment may also be produced as a hybrid protein that can be cleaved if desired. In general, host-cell-specific sequences improving the production yield of truncated KGF and truncated KGF polypeptide derivatives will be used and appropriate control sequences will be added to the expression vector, such as enhancer sequences, polyadenylation sequences, and ribosome binding sites. Once the appropriate coding sequence is isolated, it can be expressed in a variety of different expression systems; for example those used with mammalian cells, baculoviruses, bacteria, and yeast. These are discussed in turn below.

i. Mammalian systems

Mammalian expression systems are known in the art. A mammalian promoter is any DNA sequence capable of binding mammalian RNA polymerase and initiating the downstream (3') transcription of a coding sequence (e.g. structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, typically located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.].

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will typically increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they typically have a broader host range. Examples include the SV40 early gene enhancer [Dijkema et al (1985) EMBO J. 4:761] and the enhancer/promoters derived from the long terminal repeat (LTR) of the Rous Sarcoma Virus [German et al. (1982b) Proc. Natl. Acad. Sci. 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2: 215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in vitro incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either in vivo or in vitro. The leader sequence fragment typically encodes a signal peptide comprised of hydrophobic amino acids which direct the secretion of the protein from the cell. The adenovirus tripartite leader is an example of a leader sequence that provides for secretion of a foreign protein in mammalian cells.

Typically, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual.

Some genes may be expressed more efficiently when introns (also called intervening sequences) are present. Several cDNAs, however, have been efficiently expressed from vectors that lack splicing signals (also called splice donor and acceptor sites) [see e.g., Gothing and Sambrook (1981) Nature 293:620]. Introns are intervening noncoding sequences within a coding sequence that contain splice donor and acceptor sites. They are removed by a process called "splicing," following polyadenylation of the primary transcript [Nevins (1983) Annu. Rev. Biochem. 52:441; Green (1986) Annu. Rev. Genet. 20:671; Padgett et al. (1986) Annu. Rev. Biochem. 55:1119; Krainer and Maniatis (1988) "RNA splicing." In Transcription and splicing (ed. B.D. Hames and D.M. Glover)].

Typically, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g., plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replication systems, thus allowing it to be maintained, for example, in mammalian cells for expression and in a procaryotic host for cloning and amplification. Examples of such mammalian-bacteria shuttle vectors include pMT2 [Kaufman et al. (1989) Mol. Cell. Biol. 9:946 and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

ii. Baculovirus systems

The polynucleotide encoding KGF can be inserted into a suitable expression vector, for example, an insect cell expression vector, and operably linked to control elements within that vector. Vector construction employs techniques which are known in the art. In particular, for the purpose of the present invention, a baculovirus expression vector is constructed substantially in accordance to Kitts *et al.*, *BioTechniques 14:* 810-817 (1993).

Briefly, a KGF expression cassette is first constructed by insertion of the $KGF_{163}$ coding sequence into a transfer vector containing a polynucleotide sequence that is homologous to a portion of the baculovirus genome (referred herein as "the baculovirus sequences") and is capable of homolgous recombination therewith. The bacuolvirus sequences contains at least an essential polynucleotide sequence, as described in more detail below. The KGF coding sequence is inserted into the transfer vector in such a manner that it is flanked on both ends by the baculovirus sequences.

The transfer vector containing the KGF coding sequence is transfected into the host insect cells together with a mutant of wild-type baculovirus that lacks an essential polynucleotide sequence necessary for production of a functional virus. In this regard, a functional virus can be produced in the host cells upon transfection when the mutant baculovirus recombines with the KGF expression cassette.

The functional baculovirus produced in this manner, therefore, incorporates the KGF expression cassette and is suitable for transfection into new host cells for production of recombinant KGF and recombinant $KGF_{des1-23}$. Alternatively, this process can be conducted with a $KGF_{des1-23}$ coding sequence instead of a $KGF_{163}$ coding sequence.

The functional baculovirus Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the truncated KGF DNA sequence into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, inter alia, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith"), and incorporated by reference.

Prior to inserting the truncated KGF DNA sequence into the baculovirus genome, the above described components, comprising a promoter, leader (if desired), coding sequence of interest, and transcription termination sequence, are typically assembled into an intermediate transplacement construct (transfer vector).

This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g., plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a procaryotic ampicillin-resistance (amp) gene and origin of replication for selection and propagation in E. coli.

Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g. structural gene) into mRNA. A promoter will have a transcription initiation region which is usually placed proximal to the 5' end of the coding sequence. This transcription initiation region typically includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Pub. Nos. 127,839 and 155,476; and the gene encoding the p10 protein Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by in vitro incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment typically encodes a signal peptide comprised of hydrophobic amino acids which direct the translocation of the protein into the endoplasmic reticulum.

After insertion of the truncated KGF DNA sequence and/or the gene encoding the expression product precursor, an insect cell host is co-transformed with the heterologous DNA of the transfer vector and the genomic DNA of wild type baculovirus -- usually by cotransfection. The promoter and transcription termination sequence of the construct will typically comprise a 2-5kb section of the baculovirus genome. Methods for introducing heterologous DNA into the desired site in the baculovirus virus are known in the art. (See Summers and Smith supra; Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91. The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 μm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, supra; Miller et al. (1989).

Recombinant baculovirus expression vectors have been developed for infection into several insect cells. For example, recombinant baculoviruses have been developed for, inter alia: Aedes aegypti , Autographa californica, Bombyx mori, Drosophila melanogaster, Spodoptera frugiperda, and Trichoplusia ni (PCT Pub. No. WO89/046699; Carbonell et al., (1985) J. Virol. 56:153; Wright (1986) Nature 321:718; Smith et al., (1983) Mol, Cell, Biol, 3:2156; and see generally, Fraser, et al, (1989) In Vitro Cell, Dev, Biol, 25:225).

Cells and cell culture media are commercially available for both direct and fusion expression of heterologous polypeptides in a baculovirus/expression system; cell culture technology is generally known to those skilled in the art. See, e.g., Summers and Smith supra.

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g., HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e. g., proteins, lipids and polysaccharides.

In order to obtain truncated KGF expression, recombinant host cells derived from the transformants are incubated under conditions which allow expression of the recombinant truncated KGF encoding sequence. These conditions will vary, dependent upon the host cell selected. However, the conditions are readily ascertainable to those of ordinary skill in the art, based upon what is known in the art.

iii. Bacterial systems

Bacterial expression techniques are known in the art. A bacterial promoter is any DNA sequence capable of binding bacterial RNA polymerase and initiating the downstream (3") transcription of a coding sequence (e.g. structural gene) into mRNA. A promoter will have a transcription initiation region which is usually placed proximal to the 5' end of the coding sequence. This transcription initiation region typically includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18: 173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (lac) [Chang et al. (1977) Nature 198:1056], and maltose. Additional examples include promoter sequences derived from biosynthetic enzymes such as tryptophan (trp) [Goeddel et al. (1980) Nuc. Acids Res. 8:4057; Yelverton et al. (1981) Nucl. Acids Res. 9:731; U.S. 4,738,921; EPO Pub. Nos. 036 776 and 121 775]. The g-laotamase (bla) promoter system [Weissmann (1981) "The cloning of interferon and other mistakes." In Interferon 3 (ed. I. Gresser)], bacteriophage lambda PL [Shimatake et al. (1981) Nature 292:128] and T5 [U. S. 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [U.S. Patent No. 4,551,433]. For example, the tac promoter is a hybrid trp-lac promoter comprised of both trp promoter and lac operon sequences that is regulated by the lac repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophase T7 RNA polymerase/promoter system is an example of a coupled promoter system [Studier et al. (1986) J. Mol. Biol. 189:113; Tabor et al. (1985) Proc Natl. Acad. Sci. 82: 1074]. In addition, a hybrid promoter can also be comprised of a bacteriophage promoter and an E. coli operator region (EPO Pub. No. 267 851).

In addition to a functioning promoter sequence, an efficient ribosome binding site is also useful for the expression of foreign genes in prokaryotes. In E. coli, the ribosome binding site is called the Shine-Dalgarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of E. coli 16S rRNA [Steitz et al. (1979) "Genetic signals and nucleotide sequences in messenger RNA" In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli" In Molecular Cloning: A Laboratory Manual.

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by in vitro incubation with cyanogen bromide or by either in vivo on in vitro incubation with a bacterial methionine N-terminal peptidase (EPO Pub. No. 219 237).

Fusion proteins provide an alternative to direct expression. Typically, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from lacZ [Jia et al. (1987) Gene 60:197]; trpE [Allen et al. (1987) J. Biotechnol. 5:93; Makoff et al. (1989) J. Gen. Microbiol, 135:11]; and Chey [EPO Pub. No. 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (e.g. ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [U.S. 4,336,336]. The signal sequence fragment typically encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either in vivo or in vitro encoded between the signal peptide fragment and the foreign gene.

DNA encoding suitable signal sequences can be derived from genes for secreted bacterial proteins, such as the E. coli outer membrane protein gene (ompA) [Masui et al. (1983), in: Experimental Manipulation of Gene Expression; Ghrayeb et al. (1984) EMBO J. 3: 2437] and the E. coli alkaline phosphatase signal sequence (phoA) [Oka et al. (1985) Proc. Natl. Acad. Sci. 82:7212]. As an additional example, the signal sequence of the alpha-amylase gene from various Bacilus strains can be used to secrete heterologous proteins from B. subtilis [Palva et al. (1982) Proc. Natl. Acad. Sci. USA 79:5582; EPO Pub. No. 244 042].

Typically, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the trp gene in E. coli as well as other biosynthetic genes.

Typically, the above described components, comprising a promoter, signal sequence (if desired), coding sequence of interest, and transcription termination sequence, are put together into expression constructs. Expression constructs are often maintained in a replicon, such as an extrachromosomal element (e.g., plasmids) capable of stable maintenance in a host, such as bacteria. The replicon will have a replication system, thus allowing it to be maintained in a procaryotic host either for expression or for cloning and amplification. In addition, a replicon may be either a high or low copy number plasmid. A high copy number plasmid will generally have a copy number ranging from about 5 to about 200, and typically about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors typically contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EPO Pub. No. 127 328). Integrating vectors may also be comprised of bacteriophage or transposon sequences.

Typically, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev, Microbiol, 32:469]. Selectable markers may also include biosynthetic genes, such as those in the histidine, tryptophan, and leucine biosynthetic pathways.

Alternatively, some of the above described components can be put together in transformation vectors. Transformation vectors are typically comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, inter alia, the following bacteria: Bacillus subtilis [Palva et al. (1982) Proc. Natl. Acad. Sci. USA 79:5582; EPO Pub. Nos. 036 259 and 063 953; PCT WO 84/04541]; Escherichia coli [Shimatake et al. (1981) Nature 292:128; Amann et al. (1985) Gene 40:183; Studier et al. (1986) J. Mol. Biol. 189:113; EPO Pub. Nos. 036 776, 136 829 and 136 907]; Streptococcus cremoris [Powell et al. (1988) Appl. Environ. Microbiol. 54: 655]; Streptococcus lividans [Powell et al. (1988) Appl. Environ. Microbiol. 54:655]; Streptomyces lividans [U. S. 4,745,056].

Methods of introducing exogenous DNA into bacterial hosts are well-known in the art, and typically include either the transformation of bacteria treated with $CaCl_2$ or other agents, such as divalent cations and DMSO. DNA can also be introduced into bacterial cells by electroporation. Transformation procedures usually vary with the bacterial species to be transformed. See e.g., [Masson et al. (1989) FEMS Microbiol. Lett. 60: 273; Palva et al. (1982) Proc. Natl. Acad. Sci. USA 79: 5582; EPO Pub. Nos. 036 259 and 063 953; PCT Publication No. WO84/04541, Bacillus], [Miller et al. (1988) Proc. Natl. Acad. Sci. 85:856; Wang et al. (1990) J. Bacteriol. 172:949, Campylobacter]; [Cohen et al. (1973) Proc. Natl. Acad. Sci. 69:2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia]; [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus]; [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infec. Immun. 32:1295; Powell et al. (1988) Appl. Environ. Microbiol. 54:655; Somkuti et al. (1987) Proc. 4th Evr. Cong. Biotechnology 1:412, Streptococcus].

iv. Yeast expression

Yeast expression systems are also known to one of ordinary skill in the art. A yeast promoter is any DNA sequence capable of binding yeast RNA polymerase and initiating the downstream (3') transcription of a coding sequence (e.g. structural gene) into mRNA. A promoter will have a transcription initiation region which is usually placed proximal to the 5' end of the coding sequence. This transcription initiation region typically includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH)(EPO Pub. No. 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO Pub. No. 329 203). The yeast PHO5 gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (U.S. 4,876,197 and U.S. 4,880,734). Other examples of hybrid promoters include promoters which consist of the regulatory sequences of either the ADH2, GAL4, GAL10, or PHO5 genes, combined with the transcriptional activation region of a glycolytic enzyme gene such as GAP or PyK (EPO Pub. No. 164 556). Furthermore, a yeast promoter can include naturally occurring promoters of non-yeast origin that have the ability to bind yeast RNA polymerase and initiate transcription. Examples of such promoters include, inter alia, [Cohen et al. (1980) Proc. Natl. Acad. Sci. USA 77: 1078; Henikoff et al. (1981) Nature 283: 835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by in vitro incubation with cyanogen bromide.

Fusion proteins provide an alternative for yeast expression systems, as well as in mammalian, baculovirus, and bacterial expression systems. Typically, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g., EPO Pub. No. 196 056. Another example is a ubiquitin fusion protein. Such a fusion protein is made with the ubiquitin region that preferably retains a site for a processing enzyme (e.g. ubiquitin-specific processing protease) to cleave the ubiquitin from the foreign protein. Through this method, therefore, native foreign protein can be isolated (see, e.g., PCT Publ. No. WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either in vivo or in vitro. The leader sequence fragment typically encodes a signal peptide comprised of hydrophobic amino acids which direct the secretion of the protein from the cell.

DNA encoding suitable signal sequences can be derived from genes for secreted yeast proteins, such as the yeast invertase gene (EPO Pub. No. 012 873; JPO Pub. No. 62,096,086) and the A-factor gene (U.S. 4,588,684). Alternatively, leaders of non-yeast origin, such as an interferon leader, exist that also provide for secretion in yeast (EPO Pub. No. 060 057).

A preferred class of secretion leaders are those that employ a fragment of the yeast alpha factor gene, which contains both a "pre" signal sequence, and a "pro" region. The types of alpha factor fragments that can be employed include the full-length pre-pro alpha factor leader (about 83 amino acid residues) as well as truncated alpha-factor leaders (typically about 25 to about 50 amino acid residues) (U.S. 4,546,083 and U. S. 4,870,008; EPO Publ. No. 324 274). Additional leaders employing an alpha-factor leader fragment that provides for secretion include hybrid alpha factor leaders made with a presequence of a first yeast, but a pro-region from a second yeast alpha factor. See e.g., PCT Publ. No. WO89/02463.

Typically, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Typically, the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a procaryotic host for cloning and amplification. Examples of such yeast-bacteria shuttle vectors include YEp24 [Botstein et al. (1979) Gene 8:17-24]; pCI/1 [Brake et al. (1984) Proc. Natl. Acad. Sci USA 81:4642-4646]; and YRp17 [Stinchcomb et al. (1982) J. Mol. Biol. 158:157]. In addition, a replicon may be either a high or low copy number plasmid. A high copy number plasmid will generally have a copy number ranging from about 5 to about 200, and typically about 10 to about 150. A host containing a high copy number plasmid will preferably have at least about 10, and more preferably at least about 20. Enter a high or low copy number vector may be selected, depending upon the effect of the vector and the foreign protein on the host. See e.g., Brake et al., supra.

Alternatively, the expression constructs can be integrated into the yeast genome with an integrating vector. Integrating vectors typically contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver et al., supra. One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Typically, extrachromosomal and integrating expression constructs may contain selectable markers to allow for the selection of yeast strains that have been transformed. Selectable markers may include biosynthetic genes that can be expressed in the yeast host, such as ADE2, HIS4, LEU2, TRP1, and ALG7, and the G418 resistance gene, which confer resistance in yeast cells to tunicamycin and G418, respectively. In addition, a suitable selectable marker may also provide yeast with the ability to grow in the presence of toxic compounds, such as metal. For example, the presence of CUP1 allows yeast to grow in the presence of copper ions [Butt et al. (1987) Microbiol. Rev. 51:351].

Alternatively, some of the above described components can be put together into transformation vectors. Transformation vectors are typically comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, inter alia, the following yeasts: Candida albicans [Kurtz, et al. (1986) Mol. Cell. Biol. 6:142]; Candida maltosa [Kunze, et al. 91985) J. Basic Microbiol. 25:141]; Hansenula polymorpha [Gleeson, et al. (1986) J. Gen. Microbiol. 132:3459; Roggenkamp et al. (1986) Mol. Gen. Genet. 202:302]; Kluyveromyces fragilis [Das, et al. (1984) J. Bacteriol. 158:1165]; Kluyveromyces lactis [De Louvencourt et al. (1983) J. Bacteriol. 154:737; Van den Berg et al. (1990) Bio/Technology 8:135]; Pichia guillerimondii [Kunze et al. (1985) J. Basic Microbiol. 25:141]; Pichia pastoris [Cregg, et al. (1985) Mol. Cell. Biol. 5:3376; U.S. 4,837,148 and U.S. 4,929,555]; Saccharomyces cerevisiae [Hinnen et al. (1978) Proc. Natl. Acad. Sci. USA 75:1929; Ito et al. (1983) J. Bacteriol. 153:163]; Schizosaccharomyces pombe [Beach and Nurse (1981) Nature 300:706]; and Yarrowia lipolytica [Davidow, et al. (1985) Curr. Genet. 10:380-471; Gaillardin, et al. (1985) Curr. Genet. 10:49].

Methods of introducing exogenous DNA into yeast hosts are well-known in the art, and typically include either the transformation of spheroplasts or of intact yeast cells treated with alkali cations. Transformation procedures usually vary with the yeast species to be transformed. See e.g., [Kurtz et al. (1986) Mol. Cell. Biol. 6:142; Kunze et al. 91985) J. Basic Microbiol. 25:141, Candida]; [Gleeson et al. 91986) J. Gen. Microbiol. 132:3459; Roggenkamp et al. (1986) Mol. Gen. Genet. 202:302, Hansenula]; [Das et al. (1984) J. Bacteriol. 158:1165; De Louvencourt et al. (1983) J. Bacteriol. 154:1165; Van den Berg et al. (1990) Bio/Technology 8:135, Kluyveromyces]; [Cregg et al. (1985) Mol. Cell. Biol. 5:3376; Kunze et al. (1985) J. Basic Microbiol. 25:141; U.S. 4,837,148 and U.S. 4,929,555, Pichia]; [Hinnen et al. (1978) Proc. Natl. Acad. Sci. USA 75:1929; Ito et al. (1983) J. Bacteriol. 153:163, Saccharomyces]; [Beach and Nurse (1981) Nature 300:706, Schizosaccharomyces]; [Davidow et al. (1985) Curr. Genet. 10:39; Gaillardin et al. (1985) Curr. Genet. 10:49, Yarrowia].

In the present invention, selectable markers, an origin of replication, and homologous host cells sequences may be included in an expression vector. A selectable marker can be used to screen for host cells that potentially contain the expression vector. Such markers include those that render a host cell resistant to drugs such as ampicillin, chloramphenicol, erythromycin, neomycin, and tetracycline. Also, the markers may include biosynthetic genes, such as those in the histidine, tryptophan, and leucine pathways that are required for growth of the host cell. Thus, when a leu(-) host cell is used a recipient in transformation with an expression vector, and leucine is absent from the media, for example, only the cells that carry a plasmid with a leu(+) gene will survive.

In the present invention, an origin of replication may be incorporated into an expression vector to allow autonomous replication in the host cell. Such origin of replication includes those that enable an expression vector to be reproduced at a high copy number in the presence of the appropriate proteins within the cell, for example the 2μ and autonomously replicating sequences, that are effective in yeast; and the origin of replication of the viral T-antigen, that is effective in COS-7 cells.

For the purpose of the present invention, expression vectors may either be integrated into the host cell genome or remain autonomous within the cell. For integration into the host genome, the expression vector herein may include polynucleotide sequences that are homologous to sequences within the host cell genome. The homologous sequences need not be linked to the expression vector.

For example, expression vectors can integrate into the CHO genome via an unattached dihydrofolate reductase gene. In yeast, it is preferred that the homologous sequences flank the expression cassette. Particularly useful homologous yeast genome sequences for the present invention are those disclosed in PCT WO90/01800, and the HIS4 gene sequences, described in Genbank, accession no. J01331.

The choice of promoter, terminator, and other optional elements of an expression vector will also depend on the host cell chosen as is known to a person of ordinary skill in the art. This invention is not dependent on the host cell selected. Convenience and the level of protein expression desired will dictate the optimal host cell. A variety of hosts for expression are known in the art and available from the American Type Culture Collection (ATCC).

For example, bacterial hosts suitable herein for expressing the KGF fragment or analog include: *Campylobacter, Bacillus, Escherichia*, *Lactobacillus, Pseudomonas, Staphylococcus,* and *Streptococcus.* Yeast hosts from the following genera may be utilized: *Candida*, *Hansenula*, *Kluyveromyces*, *Pichia*, *Saccharomyces, Schizosaccharomyces*, and *Yarrowia*. Immortalized mammalian cells that may be used as hosts herein include CHO cells, HeLa cells, baby hamster kidney ("BHK") cells, monkey kidney cells ("COS"), and human hepatocellular carcinoma cells, e.g., Hep G2. A number of insect cell hosts are also suitable for expression of the KGF fragment or analog, including: *Aedes aegypti* , *Autographa californica*, *Bombyx mori*, *Drosophila melanogaster*, and *Spodoptera frugiperda* as described in PCT WO 89/046699; Carbonell *et al*., *J. Virol*. 56:153 (1985); Wright *Nature 321*:718 (1986); Smith *et al*., *Mol. Cell. Biol. 3*:2156 (1983); and generally, Fraser, *et al. in vitro Cell. Dev. Biol. 25*:225 (1989).

The expression vector containing the KGF fragment or analog is inserted into the host cell. Any transformation techniques that are known in the art can be used for inserting expression vectors into the host cells. For example, for transformation of bacterial hosts typically includes first treating the bacteria with either $CaCl_2$ or other agents, such as divalent cations and DMSO and allowing the exogenous DNA to be introduced to [WHAT??] with the treated bacterial cells. DNA can also be introduced into bacterial cells by electroporation or viral infection. Transformation procedures for bacterial hosts that can be used herein include those described in Masson *et al*. FEMS Microbiol. Lett. 60:273 (1989); Palva *et al*. Proc. Natl. Acad. Sci. USA 79:5582 (1982); EP Publ. Nos. 036 259 and 063 953; PCT WO 84/04541, *Bacillus*), Miller *et al*. Proc. Natl. Acad. Sci. 85:856 (1988); Wang *et al*. J. Bacteriol. 172:949 (1990), *Campylobacter*), Cohen *et al*. Proc. Natl. Acad. Sci. 69:2110 (1973); Dower *et al*. Nucleic Acids Res. 16:6127 (1988); Kushner "An improved method for transformation of *Escherichia coli* with ColE1-derived plasmids in Genetic Engineering: Proceedings of the International Symposium on Genetic Engineering (eds. H.W. Boyer and S. Nicosia) (1978); Mandel *et al*. J. Mol. Biol. 53:159 (1970); Taketo Biochim. Biophys. Acta 949:318 (1988); Escherichia, Chassy *et al*. FEMS Microbiol. Lett. 44:173 (1987) *Lactobacillus*; Fiedler *et al*. Anal. Biochem 170:38 (1988), *Pseudomonas;* Augustin *et al*. FEMS Microbiol. Lett. 66:203 (1990), *Staphylococcus,* Barany *et al*. J. Bacteriol. 144:698 (1980); Harlander "Transformation of *Streptococcus lactis* by electroporation," in Streptococcal Genetics (ed. J. Ferretti and R. Curtiss III) (1987); Perry *et al*. Infec. Immun. 32:1295 (1981); Powell *et al*. Appl. Environ. Microbiol. 54:655 (1988); Somkuti *et al*. Proc. 4th Evr. Cong. Biotechnology 1:412 (1987), *Streptococcus.*

Transformation methods for yeast hosts are well-known in the art, and typically include transformation of either spheroplasts or intact yeast cells treated with alkali cations. Yeast hosts can also be transformed by electroporation as described in *Methods in Enzymology*, Volume 194, 1991, "Guide to Yeast Genetics and Molecular Biology." For the present invention, the transformation procedure to apply vary with the yeast species to be transformed and include those described in Kurtz *et al*. Mol. Cell. Biol. 6:142 (1986); Kunze *et al*. J. Basic Microbiol. 25: 141 (1985); *Candida*; Gleeson *et al*. J. Gen. Microbiol. 132:3459 (1986); Roggenkamp *et al*. Mol. Gen. Genet. 202:302 (1986); *Hansenula*; Das *et al*. J. Bacteriol. 158:1165 (1984); De Louvencourt *et al*. J. Bacteriol. 154:1165 (1983); Van den Berg *et al*. Bio/Technology 8:135 (1990); *Kluyveromyces*; Cregg *et al*. Mol. Cell. Biol. 5:3376 (1985); Kunze *et al*. J. Basic Microbiol. 25:141 (1985); U.S. Patent Nos. 4,837,148 and 4,929,555; *Pichia*; Hinnen *et al*. Proc. Natl. Acad. Sci. USA 75;1929 (1978); Ito *et al*. J. Bacteriol. 153:163 (1983) *Saccharomyces;* Beach and Nurse Nature 300:706 (1981); *Schizosaccharomyces;* Davidow *et al*. Curr. Genet. 10:39 (1985); Gaillardin *et al*. Curr. Genet. 10:49 (1985); *Yarrowia*.

Methods for introducing heterologous polynucleotides into mammalian cells are known in the art and include, for example, viral infection, dextran-mediated transfection, calcium phosphate precipitation, polybrene mediated transfection, protoplast fusion, electroporation, encapsulation of the polynucleotide(s) in liposomes, and direct microinjection of the DNA into nuclei.

Methods for introducing heterologous DNA into the baculovirus virus to form an expression vector and for transformation of an insect host cell are also known in the art as described in Smith *et al*., Mol. Cell. Biol. 3:2156 (1983); and Lucklow and Summers, Virology 17:31 (1989). For example, the KGF fragment DNA can be inserted into the polyhedron gene by homologous double crossover recombination. Insertion can also be made at a restriction enzyme site engineered into the desired baculovirus gene, as described in Miller *et al*., Bioessays 4:91 (1989). The DNA sequence of the KGF fragment when cloned in place of the polyhedron gene in the expression vector, is flanked both 5' and 3' by polyhedron-specific sequences and is positioned downstream of the polyhedron promoter.

In the embodiment of the present invention, the newly formed baculovirus expression vector can be subsequently packaged into an infectious recombinant baculovirus. In the baculovirus expression system, homologous recombination occurs at low frequency, between about 1% and about 5%. Thus, usually, the majority of the virus produced after transfection is still wild-type virus. Recombinant viruses, however, can be identified by known methods. For example, the native virus produces polyhedron protein at very high levels in the nuclei of infected cells during a late stage of viral infection. Accumulated polyhedron protein forms occlusion bodies that also contain embedded viral particles. These occlusion bodies, up to 15 μm in size, are highly refractile, giving them a bright shiny appearance that is readily visualized under the light microscope. Cells infected with recombinant viruses lack occlusion bodies. Recombinant virus and wild-type virus can be distinguished by plating, the transfection supernatant or dilutions thereof is onto a monolayer of insect cells by standard techniques. The plaques can then be screened under the light microscope for the presence, indicative of wild-type virus, or absence, indicative of recombinant virus, of occlusion bodies as described in "Current Protocols in Microbiology" Vol. 2 (Ausubel *et al.* eds) at 16.8 (Supp. 10, 1990).

Immunoassays and activity assays that are know in the art can be utilized to determine if the transformed host cells herein are expressing the desired KGF fragment. For example, an immunofluorescence assay can be performed on the transformed host cells without separating the KGF fragments from the cell membrane. In this assay, the host cells are first fixed onto a solid support, such as a microscope slide or microtiter well. Next, the fixed host cells are exposed to an anti-KGF antibody. Preferably, to increase the sensitivity of the assay, the fixed cells are exposed to a second antibody, that is labelled and binds to the anti-KGF antibody. For instance, the secondary antibody may be labelled with an fluorescent marker. The host cells which express the KGF fragments will be fluorescently labelled can be visualized under the microscope.

## Example 1

A human KGF cDNA was inserted into a baculovirus expression system by cloning the cDNA into pAcC13Pst/Not 1 expression vector as shown in FIG. 1. This plasmid was derived from pAcC12, as described in Munemitsu *et al. Mol. Cell. Biol.* 10:5977-5982 (1990). PCR oligonucleotide primers were designed for the truncated 18 kd KGF based upon the available N-terminal amino acid sequence of KGF as described in Finch *et al. Science* 2:752-755 (1989). Flanking restriction sites (*Pst*I and *Not*I) were incorporated into the primers to facilitate subcloning into the pAcC13 expression vector, a derivative of the pVL941 transfer vector as described in Luckow *et al. Virology 170*:31-39 (1989) and Quilliam *et*

*al. Mol. Cell. Biol. 10*:290102908 (1990). This expression vector was constructed by insertion of the KGF-encoding fragment into the *Pst*I/*Not*I polylinker site of pAcC13. The KGF cDNA encoded the mature processed form of KGF, which was composed of 163 amino acid residues, with a potential N-glycosylation site at residues 14 to 16.

## Preparation of condition medium

*Spodoptera Frugiperda*, Sf9 insect cells, were infected with baculovirus, *Autographa Californica*, containing cDNA for $KGF_{1-163}$. After being diluted to [X x $10^x$] cells/ml, the cells were cultured for 48 to 72 hrs in Excell-400 in the absence of serum supplement, antibiotics, or fungicides. Culture fluid was collected and centrifuged at 10,000 x g for 30 minutes to remove floating cells and other cell debris. The condition medium was then filtered with an 0.8 μ-filter (Millipore) and approximately 5 liters of this media was concentrated to 200 ml using a filtron cassette system (Omega membrane) having a 3-kDa molecular weight cut-off.

After concentration, the conditioned medium from the transfected Sf9 cells were collected by centrifugation at 10,000 x g for 20 minutes and the medium was purified by sequential heparin Sepharose ("HS") affinity chromatography. In one embodiment of the invention, approximately 1 liter of Sf9 cells conditioned medium was ultra-filtered to produce 200 ml of ultrafiltration retentate, using an Omega membrane (Filtron) having a cut-off of 3-kDa.

## HSAC and Mono S cation exchange chromatography

The supernatant was adjusted to pH 7.2. A 30 ml sample was loaded onto a heparin Sepharose resin that had been equilibrated in 10 mM Tris-HCl pH 7.3, 150 mM NaCl. The resin was washed extensively with equilibration buffer until the absorbance had returned to baseline and was then eluted stepwise with increasing NaCl concentrations from 0.45 M to 1 M and 2 M NaCl. Aliquots were removed from the fractions for use in cell proliferation assays and 1 M NaCl fractions with the highest bioactivity were pooled. the pooled fractions were diluted five-fold with 10 mM Tris pH 7.2 (final salt concentration 0.2 M NaCl), and the sample was applied with a Super loop onto a Mono S column linked to a FPLC system (Pharmacia, Piscataway, NJ). Elution was achieved with a linear gradient (10 mM Tris pH 7.3, 0.2 M NaCl to 10 mM Tris pH 7.3, 1 M NaCl). After fraction the aliquots were tested for bioactivity, the active fractions were pooled.

The retentate was adjusted to pH 7.3 and loaded onto a HS column containing approximately a 30 ml bed volume. The column was allowed to run for approximately 2 hours at 4°C. The column was washed with 150 ml of equilibration buffer containing 10 mM Tris-HCl and 0.15 M NaCl at pH 7.3. The retained protein was eluted with 0.45 M NaCl and 1 M NaCl. The flow rate of the column during elution was adjusted to approximately 90 ml/hr and 3 ml size fractions were collected.

The bioactive 1 M NaCl fractions of the HS affinity chromatography steps were pooled and diluted 5-fold with 10 mM Tris pH 7.3 and directly loaded on a Mono S HR 5/5 column (Pharmacia). The retained proteins were eluted using a 0.2 M NaCl to 1 M NaCl gradient. Bioassays of the active fraction was determined using the Balb-Mk cell line. SDS PAGE analysis of the bioactive protein fractions isolated by Mono S cation exchange chromatography was performed. Fractions 37-39 and fractions 41-42 were pooled and 10 ul aliquots were added to SDS and 10 mM DTT. These samples were heat denatured and electrophoresed in a 12% polyacrylamide gel which was subsequently silver stained. A similar migration pattern was observed whether the samples were run in a reduced environment or a non-reduced environment. 25 Kd was the apparent molecular weight of the protein contained in fractions 37-39 and 18 kd was the apparent molecular weight of the protein contained in fractions 41-42.

The chromatogram of the HS bioactive fraction shows the profile of Balb/Mk bioactivity covering fractions 31 to 47 demonstrating the presence of 2 molecular species, one eluting at 0.55 M NaCl, and the other eluting at 0.6 M NaCl.

### KGF activity determination

The presence of KGF activity in the obtained fractions was assessed by the ability of the fractions to promote the growth of BALB/C-Mk cells. In this regard, 10 μl aliquots of certain fractions were diluted in 1 ml of 0.2 % gelatin in phosphate buffered saline ("PBS"), and 10 μl of the diluted fractions were tested for growth stimulatory activity of BALB/C-Mk cells seeded in 12-well cluster plates, each containing 22 mm wells, at $5 \times 10^3$ cells per well. All of the KGF activity was found to be retained in the column and was eluted with 1 M NaCl.

The KGF bioactive fractions eluted from the HS column with 1 M NaCl were further purified by cation exchange FPLC column chromatography. These fractions were pooled, diluted 5-fold with 10 mM Tris, at pH 7.3, and directly loaded on a Mono S HR 5/5 column from Pharmacia.

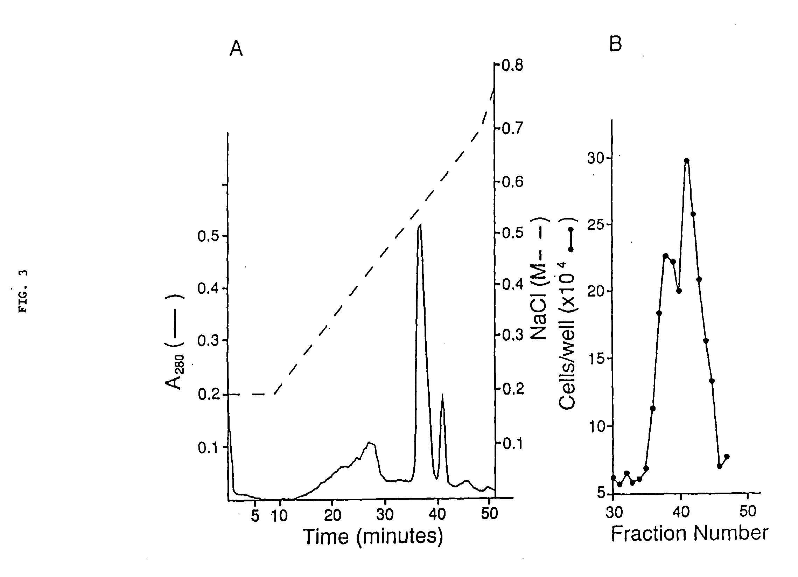

The protein retained in the Mono S HR 5/5 column was eluted with a 0.2 M NaCl to 1 M NaCl gradient. The bioactivity of the eluted fractions was assayed as described as above using BALB/C-Mk cells. Figure 3 illustrates the activity profile covering fractions 31 to 47.

In the mitogenic assay, $10_4$ ABAE and $5 \times 10_3$ ACE cells per well were seeded in 12 well cluster plates at a density of $5 \times 10^3$ cells per well in 1 ml DMEM supplemented with 10% calf serum and antibiotics as described Bohlen *et al*. *EMBO 4*:1951-1956 (1985), and Gospodarowicz *et al*. *J. Cell Physiol 127*:121-136 (1986), and Bellosta *et al*. *J. Cell Biol. 121*:705-713 (1993). After a six hour incubation, a set of triplicate wells were trypsinized and the cells were counted to determine the plating efficiency. Ten microliter aliquots of the appropriate dilution of each sample were then added in triplicate to wells in the dishes on day 0, day 2, and day 4. After the 5th day in culture, the plates were trypsinized and cell densities were determined with a Coulter counter (Coulter Electronics, Hialeah, FL).

Addition of KGF or basic FGF were done on day 0 and every other day at the indicated concentration. After 5 days in culture, the cells were trypsinized and final cell density was determined using a Coulter counter.

### Electrophoresis (NaDodSO$_4$/PAGE).

Polyacrylamide gels were prepared with NaDodSO$_4$ following the procedure as described in Laemmli *et al*. *Nature 227*:680-685 (1970). Samples were boiled for 3 min in the presence of 10 mM DTT and electrophoresed in a 12% polyacrylamide gel. The gels were fixed and silver stained using the reagents and protocol from BioRad and as discussed in Merril *et al*. *Science 211*:1437-1438. Appropriate molecular weight markers were from Biorad.

### Cell proliferation assay

The mitogenic activity of column fractions and purified samples were determined by using Balb/Mk cells as target cells. Stock cultures were grown and maintained in low calcium modified Eagle medium supplemented with 10% FCS, 50 μg/ml gentamicin, 0.25 μg/ml fungizone, and 10 ng/ml aFGF as described in Gospodarowicz *et al*. *J. Cell Physiol. 142*:325-333. Mitogenic assays cells were seeded in 12 wells cluster plates at a density of $5 \times 10^3$ to $1 \times 10^4$ cells per well in 1 ml low calcium MEM supplemented with 10% FCS as set forth in Gospodarowicz *et al*. Sample additions and final cell density determinations were made after 5 days in culture was performed as described in Gospodarowicz *et al*.

The mitogenic activity of the final purified material was tested on adult bovine aortic endothelial cells ("ABAE") and adrenal cortex-derived capillary endothelial cells ("ACE cells") as described in Gospodarowicz *et al*. *Proc. Natl. Acad. Sci. 73*:4120-4124 (1976). Stock cultures were maintained in the presence of DMEM supplemented with 10% CS, 50 μg/ml gentamicin, and 0.25 μg/ml. Fungizone was passaged weekly on gelatinized tissue culture dishes at a split ratio of 1:10.

Using standard methodology well known in the art, an unambiguous amino acid sequence was established for position 1 to 20 from the NH$_2$ terminus of KGF$_{des1-23}$ as follows:

$$S_1 \, Y \, D \, M_5 \, E \, G \, G \, D \, I \, R \, V \, R \, R \, L \, F \, X \, R \, T \, Q$$

The present invention also includes DNA segments encoding $KGF_{des1-23}$ and other shorter form of KGF lacking the unconserved $NH_2$ terminal characteristic of $KGF_{163}$ versus other members of the FGF family.

**Protein microsequencing**

Two nominal 100 picomole samples of the $KGF_{163}$ and $KGF_{des1-23}$ were analyzed by Edman degradation and AA analysis after centrifugal adsorption to polyvinylidene difluoride (PVDF, Applied Biosystems Biospin). The samples were loaded onto an Applied Biosystems 477A gas-phase protein sequenator. Twenty rounds of Edman degradation were carried out using standard software and chemicals supplied by Applied Biosystems, and identifications of PTH amino acids were made with an automated on-line HPLC column (model 120A, Applied Biosystems).

**Claims**

1. An isolated polypeptide, wherein said polypeptide is (a) a fragment of mature, full-length, human keratinocyte growth-factor (KGF) as depicted in Figure 1 or (b) an analog of said fragment, wherein said polypeptide exhibits an increase in bioactivity relative to said mature, full-length, human KGF as determined by the Balb/MK bioactivity assay.

2. A polypeptide according to claim 1, wherein said fragment has an amino acid sequence comprising the contiguous amino acid sequence of amino acids 24-163 of Figure 1 or wherein said analog is an analog of a fragment having an amino acid sequence comprising the contiguous amino acid sequence of amino acids 24-163 of Figure 1.

3. A polypeptide according to claim 1, wherein said fragment has an amino acid sequence having an N-terminus at a position between amino acid 9 and 26, inclusive, and extending to amino acid 163 of mature, full-length, human KGF as depicted in Figure 1, or wherein said analog has the same number of amino acids as a fragment having an amino acid sequence having an N-terminus as a position between amino acid 9 and amino acid 26, inclusive, and extending to amino acid 163 of mature, full-length, human KGF as depicted in Figure 1.

4. A polypeptide according to any one of the preceding claims, wherein said analog has at least 80% sequence identity to said fragment.

5. A polypeptide according to claim 4, wherein said analog has at least 90%, at least 95% or at least 98% sequence identity to a fragment of the amino acid sequence of amino acids 1-163 of Figure 1.

6. A polypeptide according to claim 4, wherein said analog has at least 90% or at least 95% sequence identity to amino acids 24-163 of Figure 1.

7. A polypeptide according to any one of the preceding claims which further comprises a methionine residue at the N-terminus.

8. A polypeptide according to any one of the preceding claims, which specifically stimulates epithelial cell proliferation.

9. A polypeptide according to any one of the preceding claims, wherein said increase in bioactivity is at least about a 2-fold increase in bioactivity.

10. A polypeptide according to claim 9, wherein said increase in bioactivity is about a 2-fold to about a 10-fold increase in bioactivity.

11. A polypeptide according to claim 2, which is glycosylated.

12. A composition comprising a polypeptide according to any one of the preceding claims and a pharmaceutically acceptable carrier.

13. A polypeptide according to any one of claims 1 to 11, for use in a method of treating a gastrointestinal disorder.

14. A polypeptide according to any one of claims 1 to 11, for use in a method for wound healing.

15. An antibody selective for a polypeptide according to any one of claims 1 to 11.

16. An isolated polynucleotide comprising a coding sequence encoding the polypeptide of any one of claims 1 to 11.

17. A non-human host cell that expresses a polynucleotide according to any one of claims 1 to 11.

18. The host cell of claim 17, which is a bacterial cell.

19. The host cell of claim 18, wherein the bacterial cell is *E. coli.*

20. A method of producing a recombinant polypeptide comprising culturing the host cell of any of claims 17 to 19 under conditions whereby the polypeptide is expressed.

FIG. 1

```
                Long Form Start                              CHO Site
                 1          5             10              ·  15
sequence        CYS-ASN-ASP-MET-THR-PRO-GLU-GLN-MET-ALA-THR-ASN-VAL-ASN-CYS-

                                              Short Form Start
                 16         20             25              30
sequence        SER-SER-PRO-GLU-ARG-HIS-THR-ARG-SER-TYR-ASP-TYR-MET-GLU-GLY-

                 31         35             40              45
sequence        GLY-ASP-ILE-ARG-VAL-ARG-ARG-LEU-PHE-CYS-ARG-THR-GLN-TRP-TYR-

                 46         50             55              60
sequence        LEU-ARG-ILE-ASP-LYS-ARG-GLY-LYS-VAL-LYS-GLY-THR-GLN-GLU-MET-

                 61         65             70              75
sequence        LYS-ASN-ASN-TYR-ASN-ILE-MET-GLU-ILE-ARG-THR-VAL-ALA-VAL-GLY-

                 76         80             85              90
sequence        ILE-VAL-ALA-ILE-LYS-GLY-VAL-GLU-SER-GLU-PHE-TYR-LEU-ALA-MET-

                 91         95             100             105
sequence        ASN-LYS-GLU-GLY-LYS-LEU-TYR-ALA-LYS-LYS-GLU-CYS-ASN-GLU-ASP-

                 106        110            115             120
sequence        CYS-ASN-PHE-LYS-GLU-LEU-ILE-LEU-GLU-ASN-HIS-TYR-ASN-THR-TYR-

                 121        125            130             135
sequence        ALA-SER-ALA-LYS-TRP-THR-HIS-ASN-GLY-GLY-GLU-MET-PHE-VAL-ALA-

                 136        140            145             150
sequence        LEU-ASN-GLN-LYS-GLY-ILE-PRO-VAL-ARG-GLY-LYS-LYS-THR-LYS-LYS-

                 151        155            160
sequence        GLU-GLN-LYS-THR-ALA-HIS-PHE-LEU-PRO-MET-ALA-ILE-THR
```

FIG. 2

Polyhedrin seq.> +1
TATAAATATtCCG---GGCGcggatcggtaccagatctgcagaattctaga

+35

KpnI      PstI      XbaI
          BglII     EcoRI

+637
ggatcctgatcagctagcagagctcgcggccgcccgggccgtaccGACTCT
BamHI      NheI          NotI   SmaI
          SacI          SfiI

FIG. 3

FIG. 4

EP 1 493 812 A2

23

FIG. 5

A.  ABAE

B.  ACE